# EUROPEAN PATENT SPECIFICATION

(11) **EP 3 623 039 B1**
(45) Date of publication and mention of the grant of the patent: **26.01.2022**
(21) Application number: 19194965.0
(22) Date of filing: 02.09.2019
(51) Int. Cl.: B01F 7/16, B01F 7/00, B01F 13/10, C12M 1/00, C12M 1/06, C12M 1/02

(54) **AGITATOR UNIT, SYSTEM AND METHOD**
RÜHRWERK, SYSTEM UND VERFAHREN
UNITÉ D'AGITATEUR, SYSTÈME ET PROCÉDÉ

(30) Priority: 13.09.2018 SE 1851081; 13.09.2018 CN 201821498440 U
(43) Date of publication of application: 18.03.2020
(73) Proprietor: BPC Instruments AB, 223 63 Lund (SE)
(72) Inventor: LIU, Jing, 226 48 LUND (SE)
(74) Representative: AWA Sweden AB

(56) References cited:
- CN-U- 205 146 261
- GB-A- 2 527 048
- JP-A- 2005 151 812
- JP-A- 2006 340 607
- US-A1- 2002 105 856
- US-A1- 2008 175 095
- US-A1- 2009 152 744

## Description

### Field of the invention

The present invention relates to an agitator unit intended for a flask.

### Technical Background

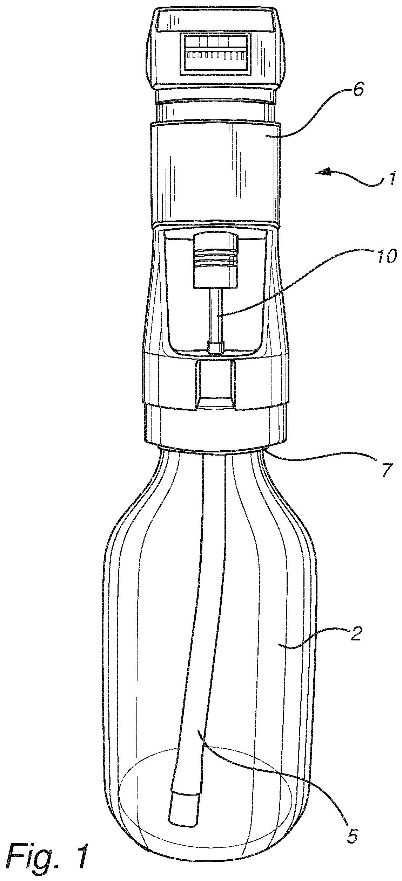

Different types of agitators are known and disclosed. One example is described in CN205146261U. CN205146261U discloses a seal reaction bottle for test of methane potentiality. The reaction bottle comprises a reaction bulb main part and an inseparable intraoral reaction bottle plug for the reaction bottle. The reaction bulb is equipped with two air ducts and a straight tube that runs through the reaction bulb. The upper end of the reaction bulb main part is equipped with a motor base, and the upper end of the motor base is equipped with the motor. The lower part of the motor is equipped with an output shaft connected with a stirring rod. The motor passes through the output shaft and drives the stirring rod to provide rotation. An agitation arrangement like the one disclosed in CN205146261U has also been published even earlier and described in the paper "An economical bioreactor for evaluating biogas potential of particulate biomass" in Bioresource Technology 92 (2004) 103-109 (see fig. 1).

One purpose of the present invention is to provide an agitator unit which is autoclavable, is easy to use and may be adapted for several types of reaction flasks and different types of industrial applications. So, the flask should be easily sealed without mounting of electrical agitator, the sealed flask should be easily autoclaved without risk for getting chemical or biological contamination during consequent experimental setup. Furthermore, it should be user friendly and convenient to set up both single and multiple flasks with agitators with little effort and time.

### Summary of the invention

The stated purpose above is achieved by an agitator unit intended for a flask, wherein the agitator unit comprises the following parts:
- an agitator rod;
- a sealing cap arranged to seal a top of the flask, said sealing cap being arranged with a through hole into which the agitator rod may be passed through, and an agitator rod tube intended to hold the agitator rod inside of the flask, wherein the sealing cap comprises one or more in/out ports;

- a motor and motor holder unit to which a connecting side or piece of the agitator rod is connectable; and
- a locking unit arranged to lock the sealing cap onto the top of the flask by screwing the locking unit onto the top of the flask;
wherein the motor and motor holder unit is connectable to the locking unit,
wherein the agitator rod tube has a closed sealing end so that the agitator rod is sealed from the inside of the flask, and wherein the agitator unit is connected to a control system enabling regulation of speed, rotation direction, time interval between clockwise and counter-clockwise movement of the agitator rod, and on/off control.

As notable from above, the agitator unit according to the present invention comprises four main parts. The agitator unit according to the present invention is simple to assemble and disassemble and still provides a safe sealing of a flask. The sealing cap is put onto a flask and the agitator rod is arranged into the sealing cap and into the agitator rod tube without being in contact with the content of the flask. A locking unit then locks everything onto the flask. Finally, the motor and motor holder unit is connected to the locking unit. The entire operation connects everything in place and seals and locks the entire agitator unit tightly together and onto the flask.

In addition to the difference of the sealing concept and other physical and hardware differences, the agitator unit according to the present invention also provides other differences when being compared to known agitators like the ones disclosed as prior art above. For example, in comparison to agitators known, the agitator unit according to the present invention provides high flexibility for a user, such as adaption for several types of reactor flasks, possibility of using different material on mixing tube and cap and auto-clavability, fluidic mixing pattern. Therefore, the agitator unit according to the present invention can meet different demands in chemical, biological and biomedical applications. Moreover, the agitator unit according to the present invention provides high user-friendliness.

The agitator unit according to the present invention provides a user-friendly assembling and dissembling feature, allows easily and good sealing of flasks without risk of chemical and biological contamination, easy experimental setup for multiple applications simultaneously without problem of wire and tube entanglement from agitators and flasks.

Moreover, the agitator unit according to the present invention provides high flexibility with reference to type, shape as well as material, and size of agitator rod. Furthermore, the agitator unit is suitable for many different size levels of flasks, however equipment intended for testing and to be used in laboratories is of large interest in relation to the present invention.

In JP 2005 151812 A there is disclosed a stirring tube made of a flexible material. A rotating shaft is rotated and driven with external power connected to the top and is deformable into a shape having a bent lower end.

Moreover, in CN 205 146 261 U there is disclosed a similar agitator unit as the one disclosed in JP 2005 151812 A.

Furthermore, in US 2008/175095 A1 a mounting and coupling assembly for a mixer is disclosed.

The device has an impeller connected to a drive shaft, which extends through an opening into the container. Moreover, the assembly includes a lip mount to stabilize and support the mixer on the lip of said container. A cover secures the lip mount with threaded fasteners engaging complementary fasteners on the container neck and clamping the plate to the container lip.

In GB 2 527 048 A there is disclosed a device for driving a plurality of rotatable agitators for use in a static batch reactor system. Furthermore, in US 2009/152744 A1 there is disclosed a reaction vessel assembly with ambient gas exchange enhancing means, said reactor vessel assembly comprising a vessel, a gas intake passage, a stirring means, and a gas venting passage.

In relation to the present invention, features and advantages of the present invention are discussed below.

### Brief description of the drawings

In figs. 1-5 there is shown an agitator unit and different parts thereof according to one embodiment of the present invention.

### Specific embodiments of the invention

Below specific embodiments of the present invention are disclosed.

According to the present invention, the agitator rod tube has a closed sealing end so that the agitator rod is sealed from the inside of the flask. This feature ensures a total seal of the content of the flask, i.e. also from the agitator rod. Moreover, this feature also provides high flexibility for a user. The user can choose different types of agitator rods, if necessary. Furthermore, to assemble the agitator unit and also insert the agitator rod is made very simple for a user.

According to yet another specific embodiment of the present invention, the agitator rod tube together with an inserted agitator rod is arranged to provide clockwise and/or counter-clockwise rotation movement which serves as a mechanical finger design agitation. This type of agitation, that is finger type agitation, according to the present invention provides agitation where the agitator rod rotates with a circular or elliptic (oval) path around a vertical line inside of the flask, and where the path is provided at a distance from the vertical line in all positions. To continue on this perspective, according to another embodiment of the present invention, the agitator rod is arranged to extend at a peripheral distance from a vertical geometrical line extending through the through hole of the sealing cap. The agitator rod may be angled on at least one position along the agitator rod or is bent. This provides a suitable finger type agitation as explained above. Moreover, the agitator rod with one angle is often a shorter version and especially suitable for smaller flask sizes in the range of 100 - 1000 ml, e.g. GL flasks, such as a typical GL 45 laboratory bottle. Again, one of the key advantages of the agitator unit according to the present invention is its simplicity of mounting on standard laboratory glass bottles. According to yet another specific embodiment of the present invention, the agitator rod is angled or bent at two different positions along the agitator rod. This may have different configurations. As an example, the two angle agitator rods may be angled or bent first upwards and then downwards again. This type of agitator design may be suitable for larger reactor bottles, e.g. in the range of from 1000 - 10000 ml. In general, the agitator unit according to the present invention finds use for flasks or bottles having a size of from 50 ml up to 10 I. The length and shape of the agitator rod may of course be adjusted accordingly.

Furthermore, according to one specific embodiment of the present invention, the agitator rod tube is flexible. This also implies that the agitator rod tube is made of a flexible material, such as suitable rubber materials. This also has benefits in relation to simplifying when inserting the agitator rod into the agitator rod tube with or with need of lubricant such as grease or detergent. The choice of rubber material may be made with reference to certain requirements regarding environmental, chemical and biotechnological applications.

Moreover, according to the present invention, the locking unit is arranged to lock the sealing cap onto the seal on the top of the flask by screwing the locking unit onto the top of the flask. It should be noted that in the case of standard flasks screwing locking is very suitable.

Furthermore, according to the present invention, the sealing cap comprises one or more in/out ports. This enables to both flow a liquid or a gas into the flask, but of course also lead produced gas away from the flask. As such, a flask having an agitator unit according to the present invention may be connected to measuring devices to measure produced gas or a flow into the flask, as well as dosing chemical into the flask etc. during the mixing processes. Moreover, as stated above, the agitator unit according to the present invention is connected to a control system enabling regulation of speed, rotation direction, time interval between clockwise and counter-clockwise movement of the agitator rod, and on/off control.

Moreover, other parts of the agitator unit may vary, but also specific alternatives may be of certain interest in some cases. As an example, according to one specific embodiment of the present invention, the agitator unit has a brushless step motor which provides not only a long lifetime and stable performance, but also high accuracy and precision control of rotation speed. This should of course only be seen as one alternative according to the present invention, and other types brush DC and AC motors may also be alternatives but they often have poor performance in comparison with a brushless step motor. Furthermore, the agitator unit according to the present invention may be equipped with and without a gear box based on application demand. Moreover, the power output of the motors may of course be chosen in order to meet the demand on torque capacity.

According to yet another specific embodiment, the present invention refers to an agitator system comprising several flasks and several agitator units according to the present invention and where the individual agitator units and flasks are connected either in series or parallel, or any hybrid mode. As an example, up to around 15-18 agitators may be connected in serial via 8-pin data and power connection cable. Such a simple data bus system allows all individual agitator to perform correctly with less risk of performance lose. Furthermore, according to yet another embodiment of such a system as above, each agitator unit is individually controllable. It should be noted that all levels of this is possible according to the present invention, i.e. from totally individual to semi-individual and also with most of things being controlled on a system level and not individually. To give some examples, the agitator system according to the present invention may in some cases not allow fully control of individual agitators. All agitators may e.g. be operated at certain rotation speeds and on/off interval. In such a case, it is possible to manually change the rotation direction and turn the individual agitators on and off. Again, all versions of levels of individual controlling are possible according to the present invention.

The present invention also refers to a method comprising using an agitator unit in a reactor flask, or using an agitator system in several rector flasks, in chemical, food, feed, agriculture, environmental, biotechnical and biomedical and clinical laboratories. As hinted above, the material of the agitator rod tube may be selected with different biological, chemical, physical and mechanical properties for these certain chemical, food, agriculture, environmental, biomedical and clinical laboratory applications.

The present disclosure also refers to a module design comprising four parts including motor holder unit, locking unit, sealing cup with agitator rod tube and agitator rod with connecting piece. The sealing cup with agitator rod tube can be pushed and set onto the top of a flask and sealed with help of locking unit without adding motor holder and agitator rod. With this arrangement on purpose, the agitator unit according to the present invention provides a user-friendly assembling and dissembling feature, allows easily and good sealing of flasks without risk of chemical and biological contamination, easy experimental setup for multiple applications simultaneously without problem of wire and tube entanglement from agitators and flasks.

As discussed above, the present invention has several advantages. The agitator unit is suitable for standard GL laboratory flasks. It is autoclavable, which supports a broad set of applications. The sealing cap of the agitator unit is user-friendly and can be easily mounted on a flask without using much force and any lubricant. Moreover, the sealing cap may be made of engineering plastic having special rubber sealing on three sides. Two gas in/out ports may be provided. Moreover, the present invention provides a multifunctional agitator unit and system which may be arranged with a brushless DC motor for long-life operation. Speed and on/off control may be provided. Moreover, it may have manually adjustable direction and time interval, as well as a timer function to set time periods for reversal. Moreover, in the agitator system modular design for low maintenance may be provided.

### Detailed description of the drawings

In fig. 1 there is shown one an agitator unit 1 according to one embodiment of the present invention, which is connected to a flask 2. The agitator unit 1 comprises an agitator rod 10. In fig. 1 there is also shown an alternative of a motor and motor holder unit 6 to which the agitator rod 10 is connected. Moreover, an agitator rod tube 5 holds the agitator rod 10 sealed inside of the flask 2 so that it does not come into physical contact with the content of the flask. Furthermore, the motor and motor holder unit 6 is connected to a locking unit 7, which in turn locks and seals the entire connection between the agitator unit 1 and the flask 2.

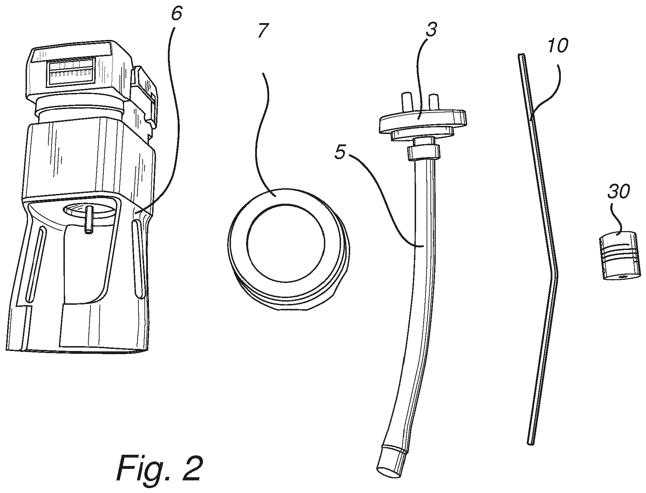

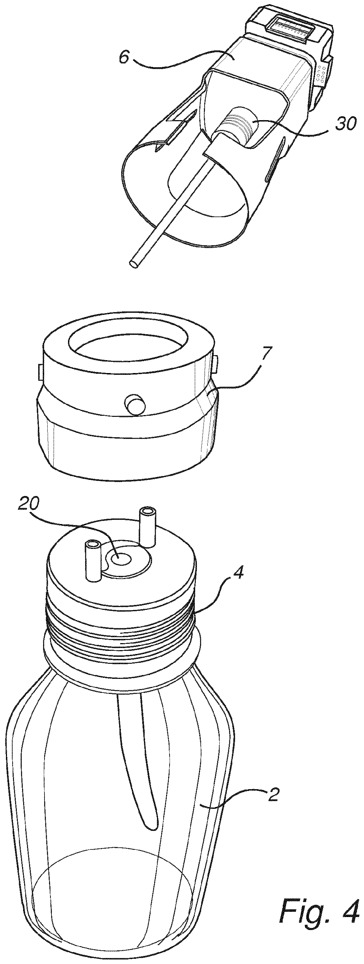

In fig. 2 all different parts are shown of an agitator unit 1 according to one embodiment of the present invention. To the far left, the motor and motor holder unit 6 is shown. To the right thereof, a locking unit 7 is shown, in this case in the form a threaded locking unit 7 which is screwed on top of a flask 2 to lock the same. Thereafter, there is shown a sealing cap 3 arranged to seal a top 4 of a flask 2. The sealing cap 3 is arranged with a through hole 20 (see an alternative in fig. 4) into which an agitator rod 10 may be passed through, and where an agitator rod tube 5 holds the agitator rod 10 inside of the flask 2 without coming into physical contact with the content of the flask 2. As notable, in this case the agitator rod 10 is angled once along the agitator rod 10. Moreover, the agitator rod 10 is connectable to a connecting side or piece 30, which in turn is connectable to the motor of the motor and motor holder unit 6 so that the agitator rod 10 may be driven and rotated.

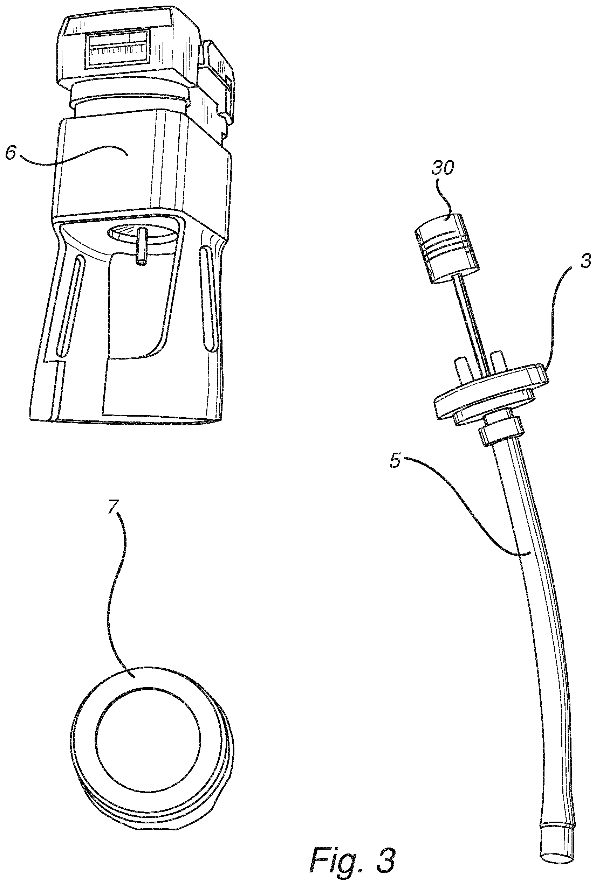

In fig. 3, the different parts according to the same embodiment as shown in fig. 1 and 2 are shown from a different view perspective. Moreover, in this case the agitator rod 10 with connected connecting piece 30 is inserted through the sealing cap 3 and into the agitator rod tube 5, which is closed in the end so that the entire unit is totally sealable when being connected to a flask 2.

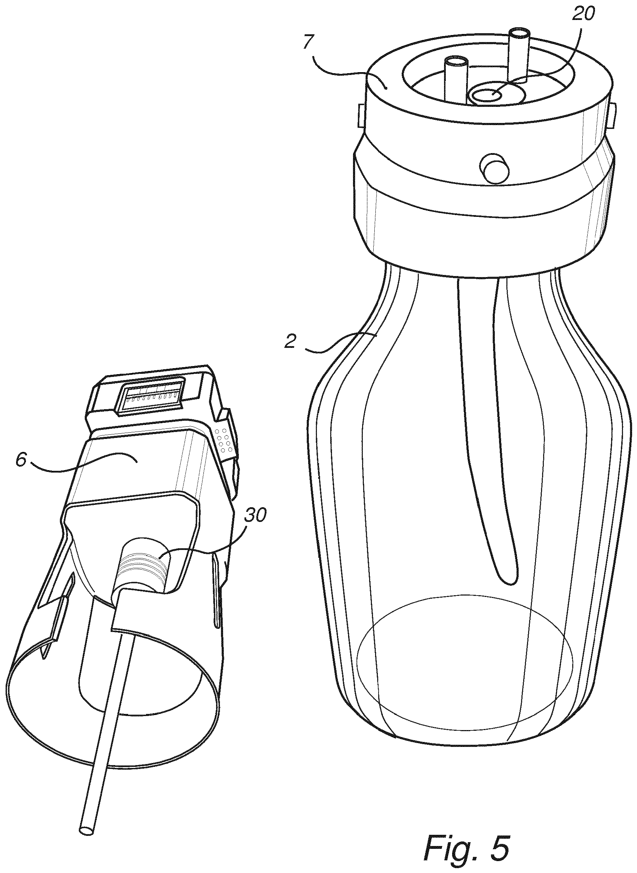

In fig. 4, then the sealing cap 3 is connected to a flask 2. Moreover, here the through hole 20 of the sealing cap 3 is shown. The locking unit 7 is arranged to lock the sealing cap 3 onto the top 4 of the flask 2. Furthermore, the motor and motor holder unit 6 is connectable to the locking unit 7. Moreover, in fig. 5 there is shown the same embodiment, however in this case the locking unit 7 is screwed to lock and seal the sealing cap 3 onto the flask 2.

## Claims

1. Agitator unit (1) intended for a flask (2), wherein the agitator unit (1) comprises the following parts:
- an agitator rod (10);
- a sealing cap (3) arranged to seal a top (4) of the flask (2), said sealing cap (3) being arranged with a through hole (20) into which the agitator rod (10) may be passed through, and an agitator rod tube (5) intended to hold the agitator rod (10) inside of the flask (2);
- a motor and motor holder unit (6) to which a connecting side or piece (30) of the agitator rod (10) is connectable; and
- a locking unit (7) arranged to lock the sealing cap (3) onto the top (4) of the flask (2);
wherein the motor and motor holder unit (6) is connectable to the locking unit (7), wherein the agitator rod tube (5) has a closed sealing end so that the agitator rod (10) is sealed from the inside of the flask (2), **characterized in that**:
the sealing cap (3) comprises one or more in/out ports;
the locking unit (7) is arranged to lock the sealing cap (3) onto the seal on the top (4) of the flask (2) by screwing the locking unit (7) onto the top (4) of the flask (2); and
the agitator unit (1) is connected to a control system enabling regulation of speed, rotation direction, time interval between clockwise and counter-clockwise movement of the agitator rod, and on/off control.

2. Agitator unit (1) according to claim 1, wherein the agitator rod tube (5) together with an inserted agitator rod (10) is arranged to provide clockwise and/or counter-clockwise rotation movement which serves as a mechanical finger design agitation.

3. Agitator unit (1) according to claim 1 or 2, wherein the agitator rod tube (5) is flexible.

4. Agitator unit (1) according to any of claims 1-3, wherein the agitator rod (10) is arranged to extend at a peripheral distance from a vertical geometrical line extending through the through hole (20) of the sealing cap (3).

5. Agitator unit (1) according to any of claims 1-4, wherein the agitator rod (10) is angled on at least one position along the agitator rod (10) or is bent.

6. Agitator unit (1) according to any of claims 1-4, wherein the agitator rod (10) is angled or bent at two different positions along the agitator rod (10).

7. Agitator unit (1) according to any of claims 1-6, wherein the agitator unit (1) has a brushless step motor.

8. Agitator system comprising several flasks (2) and several agitator units (1) according to any of claims 1-7, wherein the individual agitator units (1) and flasks (2) are connected in either series or parallel, or any hybrid mode.

9. Agitator system according to claim 8, wherein each agitator unit (1) is individually controllable.

10. Method comprising using an agitator unit (1) according to any of claims 1-7 in a reactor flask (2), or using an agitator system according to claim 8 or 9 in several rector flasks (2), in chemical, food, feed, agriculture, environmental, biotechnological, biomedical and clinical laboratories.

## Patentansprüche

1. Rührwerk (1) für einen Kolben (2), wobei das Rührwerk (1) die folgenden Teile umfasst:
- einen Rührstab (10);
- eine Dichtungskappe (3), die angeordnet ist, um eine Oberseite (4) des Kolbens (2) abzudichten, wobei die Dichtungskappe (3) mit einem Durchgangsloch (20), durch das der Rührstab (10) hindurchgeführt werden kann, und einem Rührstabrohr (5), das den Rührstab (10) im Inneren des Kolbens (2) halten soll, angeordnet ist;
- eine Motor- und Motorhalteeinheit (6), mit der eine Verbindungsseite oder ein Verbindungsteil (30) des Rührstabs (10) verbindbar ist; und
- eine Arretiereinheit (7), die angeordnet ist, um die Dichtungskappe (3) an der Oberseite (4) des Kolbens (2) zu arretieren;
wobei die Motor- und Motorhalteeinheit (6) mit der Arretiereinheit (7) verbindbar ist, wobei das Rührstabrohr (5) ein geschlossenes Dichtungsende aufweist, sodass der Rührstab (10) gegenüber dem Inneren des Kolbens (2) abgedichtet ist, **dadurch gekennzeichnet,**
**dass**
die Dichtungskappe (3) eine oder mehrere Einlass-/Auslassöffnungen umfasst;
die Arretiereinheit (7) angeordnet ist, um die Dichtungskappe (3) auf der Dichtung an der Oberseite (4) des Kolbens (2) durch Aufschrauben der Arretiereinheit (7) auf die Oberseite (4) des Kolbens (2) zu arretieren; und
das Rührwerk (1) mit einem Steuersystem verbunden ist, das eine Regulierung der Geschwindigkeit, der Drehrichtung, des Zeitintervalls zwischen der Bewegung des Rührstabs im und gegen den Uhrzeigersinn sowie eine Ein-/Ausschaltsteuerung ermöglicht.

2. Rührwerk (1) nach Anspruch 1, wobei das Rührstabrohr (5) zusammen mit einem eingesetzten Rührstab (10) angeordnet ist, um eine Drehbewegung im und/oder gegen den Uhrzeigersinn bereitzustellen, die als eine mechanische Fingerdesign-Rührbewegung dient.

3. Rührwerk (1) nach Anspruch 1 oder 2, wobei das Rührstabrohr (5) flexibel ist.

4. Rührwerk (1) nach einem der Ansprüche 1 - 3, wobei der Rührstab (10) so angeordnet ist, dass er sich in einem Umfangsabstand von einer vertikalen geometrischen Linie erstreckt, die durch das Durchgangsloch (20) der Dichtungskappe (3) verläuft.

5. Rührwerk (1) nach einem der Ansprüche 1 - 4, wobei der Rührstab (10) an zumindest einer Position entlang des Rührstabs (10) abgewinkelt oder gebogen ist.

6. Rührwerk (1) nach einem der Ansprüche 1 - 4, wobei der Rührstab (10) an zwei unterschiedlichen Positionen entlang des Rührstabs (10) abgewinkelt oder gebogen ist.

7. Rührwerk (1) nach einem der Ansprüche 1 - 6, wobei das Rührwerk (1) einen bürstenlosen Schrittmotor aufweist.

8. Rührsystem, umfassend mehrere Kolben (2) und mehrere Rührwerke (1) nach einem der Ansprüche 1 - 7, wobei die einzelnen Rührwerke (1) und Kolben (2) in Reihe, parallel oder in einer beliebigen Mischform geschaltet sind.

9. Rührsystem nach Anspruch 8, wobei jedes Rührwerk (1) einzeln steuerbar ist.

10. Verfahren, umfassend die Verwendung eines Rührwerks (1) nach einem der Ansprüche 1 - 7 in einem Reaktionskolben (2) oder die Verwendung eines Rührsystems nach Anspruch 8 oder 9 in mehreren Reaktionskolben (2) in Chemie-, Lebensmittel-, Futtermittel-, Landwirtschafs-, Umwelt-, biotechnologischen, biomedizinischen und klinischen Laboratorien.

## Revendications

1. Unité d'agitateur (1) destinée à un flacon (2), l'unité d'agitateur (1) comprenant les parties suivantes :
une tige d'agitateur (10) ;
un bouchon d'étanchéité (3) conçu pour fermer hermétiquement une partie supérieure (4) du flacon (2), ledit bouchon d'étanchéité (3) étant conçu avec un trou traversant (20) au travers duquel la tige d'agitateur (10) peut être passée, et un tube de tige d'agitateur (5) destiné à maintenir la tige d'agitateur (10) à l'intérieur du flacon (2) ;
une unité moteur et support de moteur (6) à laquelle une face ou une pièce de liaison (30) de la tige d'agitateur (10) peut être reliée ; et
une unité de verrouillage (7) conçue pour verrouiller le bouchon d'étanchéité (3) sur la partie supérieure (4) du flacon (2) ;
l'unité moteur et support de moteur (6) pouvant être reliée à l'unité de verrouillage (7), le tube de tige d'agitateur (5) ayant une extrémité étanche fermée de sorte que la tige d'agitateur (10) soit rendue étanche par rapport à l'intérieur du flacon (2), **caractérisée en ce que** :
le bouchon d'étanchéité (3) comprend un ou plusieurs orifices d'entrée/de sortie ;
l'unité de verrouillage (7) est conçue pour verrouiller le bouchon d'étanchéité (3) sur le joint d'étanchéité sur la partie supérieure (4) du flacon (2) en vissant l'unité de verrouillage (7) sur la partie supérieure (4) du flacon (2) ; et
l'unité d'agitateur (1) est reliée à un système de commande permettant de réguler la vitesse, le sens de rotation, l'intervalle de temps entre le mouvement dans le sens horaire et antihoraire de la tige d'agitateur, et la commande marche/arrêt.

2. Unité d'agitateur (1) selon la revendication 1, le tube de tige d'agitateur (5) avec une tige d'agitateur insérée (10) étant conçu pour fournir un mouvement de rotation dans le sens horaire et/ou antihoraire qui sert d'agitation de type à doigt mécanique.

3. Unité d'agitateur (1) selon la revendication 1 ou 2, le tube de tige d'agitateur (5) étant flexible.

4. Unité d'agitateur (1) selon l'une quelconque des revendications 1 à 3, la tige d'agitateur (10) étant conçue pour s'étendre au niveau d'une distance périphérique à partir d'une ligne géométrique verticale s'étendant à travers le trou traversant (20) du bouchon d'étanchéité (3).

5. Unité d'agitateur (1) selon l'une quelconque des revendications 1 à 4, la tige d'agitateur (10) étant inclinée sur au moins une position le long de la tige d'agitateur (10) ou étant pliée.

6. Unité d'agitateur (1) selon l'une quelconque des revendications 1 à 4, la tige d'agitateur (10) étant inclinée ou pliée en deux positions différentes le long de la tige d'agitateur (10).

7. Unité d'agitateur (1) selon l'une quelconque des revendications 1 à 6, l'unité d'agitateur (1) ayant un moteur pas à pas sans brosse.

8. Système d'agitateur comprenant plusieurs flacons (2) et plusieurs unités d'agitateur (1) selon l'une quelconque des revendications 1 à 7, les unités d'agitateur (1) individuelles et les flacons (2) étant reliés en série ou en parallèle, ou selon n'importe quel mode hybride.

9. Système d'agitateur selon la revendication 8, chaque unité d'agitateur (1) pouvant être commandée individuellement.

10. Procédé comprenant l'utilisation d'une unité d'agitateur (1) selon l'une quelconque des revendications 1 à 7 dans un flacon de réactif (2), ou l'utilisation d'un système d'agitateur selon la revendication 8 ou 9 dans plusieurs flacons de réactif (2), dans des laboratoires chimiques, alimentaires, d'alimentation animale, agricoles, environnementaux, biotechnologiques, biomédicaux et cliniques.
